# EUROPEAN PATENT APPLICATION

(11) **EP 3 960 161 A1**
(43) Date of publication of application: **02.03.2022**
(21) Application number: 21193093.8
(22) Date of filing: 25.08.2021
(51) Int. Cl.: A61K 9/16, A61K 9/20, A61K 31/609, A61K 31/167

(54) **POLYVINYL ALCOHOL-CONTAINING GRANULATED PRODUCT AND SOLID PREPARATION**

(30) Priority: 28.08.2020 JP 2020144742
(71) Applicant: Shin-Etsu Chemical Co., Ltd., Tokyo 100-0004 (JP)
(72) Inventor: HIRAMA, Yasuyuki, NIIGATA, 942-8601 (JP)
(74) Representative: Nony

(57) **Abstract**

There are provided a granulated product (GP) including at least one selected from an active ingredient, an excipient and a disintegrant, and 0.1 to 5.0% by mass, based on mass of the GP, of a polyvinyl alcohol having a saponification degree of from 60 to 77 mol%; a solid preparation including the GP; and an active ingredient in case where the GP does not contain the active ingredient; and a method for producing a GP, including a granulation step of granulating, while adding a second component including at least water, a first component including at least one selected from an active ingredient, an excipient and a disintegrant to obtain the GP, wherein the first component and/or the second component includes a polyvinyl alcohol having a saponification degree of 60 to 77 mol% in an amount of from 0.1 to 5.0% by mass, based on mass of the GP.

## Description

### BACKGROUND OF THE INVENTION

### 1. Field of the Invention

The invention relates to a granulated product containing a polyvinyl alcohol, and a solid preparation containing the granulated product and exceling in disintegration in the field of pharmaceuticals or health foods.

### 2. Related Art

A dosage form for oral administration of active ingredient in the field of pharmaceuticals and health foods such as a food with nutrient function claims includes a solid preparation such as a tablet, granules and a capsule; and a liquid preparation such as an elixir, a suspension and an emulsion. Of these, the tablet is a solid preparation obtained by tableting powder into a constant shape, and has advantages such as easy handling and easy taking. Especially in the field of pharmaceuticals, a tablet accounts for about 50% of the total production value and is most commonly used.

Examples of the method for producing a tablet include a dry direct tableting, a dry granulation tableting, an extrusion granulation tableting, and a wet granulation tableting. The wet granulation tableting is more complicated than the dry direct tableting in which, for example, a drug and an excipient are mixed and tableted as they are. However, the wet granulation tableting is more commonly used because the compressibility, flowability and uniformity of drug content can be greatly improved by the granulation operation. The wet granulation tableting is a method in which a mixture of, for example, a drug and an excipient, is granulated by utilizing adhesion of a binder solution or a solvent such as water; dried; and the obtained granulated product is tableted to produce a tablet. The wet granulation tableting includes a wet stirring granulation tableting using a stirring granulator, and a fluidized bed granulation tableting using a fluidized bed granulator.

A solid preparation in which the obtained granulated product is orally administered as a pharmaceutical product or a health food without tableting is referred to as granules. A solid preparation obtained by filling a capsule with a granulated product is referred to as a capsule.

Main examples of the binder to be used during the granulation in the wet granulation tableting include polyvinyl pyrrolidone (hereinafter also referred to as "PVP"), hydroxypropyl cellulose (hereinafter also referred to as "HPC") and hydroxypropyl methyl cellulose (hereinafter also referred to as "HPMC").

However, the binding strength of said binder may be insufficient so that a granulated product having a predetermined particle size may not be obtained, a tablet having desirable tablet hardness may not be obtained, or a tableting disorder such as capping may occur during tableting. When the amount of said binder is increased to enhance the binding strength, there is a problem that the disintegration deteriorates.

Therefore, it is desired to develop a granulated product which has a sufficient particle size without increasing the amount of the binder, and which can provide a tablet having high tablet hardness and a short disintegration time when tableted; and a method for producing the granulated product without using a special technique or apparatus.

When PVP, HPC or HPMC results in lack of binding strength, a polyvinyl alcohol (hereinafter also referred to as "PVA") may be used as a binder with high binding strength. For example, a granulated product obtained through a wet stirring granulation by using PVA which conforms to the Japanese Pharmaceutical Excipients is known to have a reduced amount of fine particles and a low degree of compaction and excel in flowability in comparison with a granulated product by using HPC or HPMC; and a tablet obtained by tableting the granulated product containing PVA is known to have higher tablet hardness and a lower friability in comparison with a tablet obtained by tableting the granulated product containing HPC or HPMC (Proceedings of 30th Symposium on Particulate Preparations and Designs, 2013, pages 38 to 39).

In order to improve solubility and dissolution of a poorly soluble drug, PVA is also used as a carrier of a solid dispersion in which a drug is amorphized and dispersed at a molecular level in a carrier (WO 2018/199281A and WO 2018/199282A)

### SUMMARY OF THE INVENTION

However, when PVA is used as a binder in a granulated product obtained through a wet granulation including a wet stirring granulation, the disintegration time may be delayed in comparison with that for usage of the other binder.

The invention is made in view of the above circumstances, and an object of the invention is to provide a granulated product excellent in disintegration even when it contains PVA as a binder; a method for producing said granulated product; and a solid preparation containing said granulated product. Since the invention relates to a granulated product, a crystalline drug does not become amorphous during production of a granulatedn product, contrary to a solid dispersion. The granulated product does not include a solid dispersion except for the case where the solid dispersion is granulated.

As a result of extensive studies to solve the above problem, the inventors have found that a tablet excellent in hardness and disintegration may be obtained by using PVA having a saponification degree of from 60 to 77 mol% during granulation in a wet granulation tableting, and completed the invention.

In an aspect of the invention, there is provided a granulated product comprising at least one selected from the group consisting an active ingredient, an excipient and a disintegrant; and 0.1 to 5.0% by mass, based on mass of the granulated product, of a polyvinyl alcohol having a saponification degree of from 60 to 77 mol%.

In another aspect of the invention, there is provided a solid preparation comprising the granulated product and, in a case where the granulated product does not contain an active ingredient, the active ingredient.

In still another aspect of the invention, there is provided a method for producing a granulated product, the method comprising a granulation step of granulating, while adding a second component comprising at least water, a first component comprising at least one selected from the group consisting of an active ingredient, an excipient and a disintegrant to obtain the granulated product, wherein the first component and/or the second component comprises a polyvinyl alcohol having a saponification degree of from 60 to 77 mol% in an amount of from 0.1 to 5.0% by mass, based on mass of the granulated product.

In a further aspect of the invention, there is provided a method for producing a tablet, the method comprising a step of tableting powder comprising the granulated product and a lubricant to obtain a tablet.

According to the invention, a high-quality solid preparation excellent in hardness and disintegration may be produced so that the occurrence of cracks and chips may be suppressed during filling, transportation or the like; and drug efficacy may be quickly exhibited when orally administered.

### DETAILED DESCRIPTION OF THE PREFERRED EMBODIMENTS

### (1) Granulated Product

The granulated product comprises at least one selected from a group consisting of an active ingredient, an excipient and a disintegrant; and 0.1 to 5.0% by mass, based on mass of the granulated product, of a polyvinyl alcohol having a saponification degree of from 60 to 77 mol% as a binder.

The active ingredient is not particularly limited as long as it is an active ingredient that can be orally administered. Examples of the active ingredient include drugs used in pharmaceutical products and active ingredients used in health foods such as foods with nutrient function claims, foods for specified health use, and foods with functional claims.

Example of the drug used in pharmaceutical products include a drug for the central nervous system, a drug for the cardiovascular system, a drug for the respiratory system, a drug for the digestive system, an antibiotic, an antitussive and expectorant, an antihistamine, an antipyretic anti-inflammatory analgesic, a diuretic, an autonomic agent, an antimalarial agent, an antidiarrheal agent, a psychotropic, and vitamins and derivatives thereof.

Examples of the drug for the central nervous system include diazepam, idebenone, naproxen, piroxicam, indomethacin, sulindac, lorazepam, nitrazepam, phenytoin, acetaminophen, ethenzamide, ketoprofen, and chlordiazepoxide.

Examples of the drug for the cardiovascular system include molsidomine, vinpocetine, propranolol, methyldopa, dipyridamole, furosemide, triamterene, nifedipine, atenolol, spironolactone, metoprolol, pindolol, captopril, isosorbide dinitrate, delapril hydrochloride, meclofenoxate hydrochloride, diltiazem hydrochloride, etilefrine hydrochloride, digitoxin, and alprenolol hydrochloride.

Examples of the drug for the respiratory system include amlexanox, dextromethorphan, theophylline, pseudoephedrine, salbutamol, and guaifenesin.

Examples of the drug for the digestive system include a benzimidazole drug having antiulcer action, such as 2-[[3-methyl-4-(2,2,2-trifluoroethoxy)-2-pyridy1]methylsulfiny1]benzimidazole and 5-methoxy-2-[(4-methoxy-3,5-dimethyl-2-pyridyl)methylsulfinyl]benzimidazole; cimetidine; ranitidine; pirenzepine hydrochloride; pancreatin; bisacodyl; and 5-aminosalicylic acid.

Examples of the antibiotic include talampicillin hydrochloride, bacampicillin hydrochloride, cefaclor, and erythromycin.

Examples of the antitussive and expectorant include noscapine hydrochloride, carbetapentane citrate, isoaminile citrate, and dimemorfan phosphate.

Examples of the antihistamine include chlorpheniramine maleate, diphenhydramine hydrochloride, and promethazine hydrochloride.

Examples of the antipyretic anti-inflammatory analgesic include ibuprofen, diclofenac sodium, flufenamic acid, sulpyrine, aspirin, and ketoprofen.

Examples of the diuretic include caffeine.

Examples of the autonomic agent include dihydrocodeine phosphate, dl-methylephedrine hydrochloride, atropine sulfate, acetylcholine chloride, and neostigmine.

Examples of the antimalarial agent include quinine hydrochloride.

Examples of the antidiarrheal agent include loperamide hydrochloride.

Examples of the psychotropic include chlorpromazine.

Examples of the vitamins and derivatives thereof include vitamin A, vitamin B1, fursultiamine, vitamin B2, vitamin B6, vitamin B12, vitamin C, vitamin D, vitamin E, vitamin K, calcium pantothenate, and tranexamic acid.

Examples of the active ingredient used in the health food include the above vitamins and derivatives thereof, minerals, carotenoids, amino acids and derivatives thereof, plant extracts, and health food materials.

Examples of the mineral include calcium, magnesium, manganese, zinc, iron, copper, selenium, chromium, sulfur, and iodine.

Examples of the carotenoid include β-carotene, α-carotene, lutein, cryptoxanthin, zeaxanthin, lycopene, astaxanthin, and multicarotene.

Examples of the amino acid include an acidic amino acid, a basic amino acid, a neutral amino acid, and an acidic amino acid amide.

Examples of the acidic amino acid include aspartic acid and glutamic acid.

Examples of the basic amino acid include lysine, arginine, and histidine.

Examples of the neutral amino acid include linear aliphatic amino acids such as alanine and glycine; branched aliphatic amino acids such as valine, leucine and isoleucine; hydroxyamino acids such as serine and threonine; sulfur-containing amino acids such as cysteine and methionine; aromatic amino acids such as phenylalanine and tyrosine; heterocyclic amino acids such as tryptophan; and imino acids such as proline.

Examples of the acidic amino acid amide include asparagine and glutamine.

Examples of the amino acid derivative include acetylglutamine, acetylcysteine, carboxymethylcysteine, acetyltyrosine, acetylhydroxyproline, 5-hydroxyproline, glutathione, creatine, S-adenosylmethionine, glycylglycine, glycylglutamine, dopa, alanylglutamine, carnitine and γ-aminobutyric acid.

Examples of the plant extract include aloe extract, propolis extract, agaricus extract, Panax ginseng extract, ginkgo leaf extract, turmeric extract, curcumin, sprouted brown rice extract, shiitake mycelium extract, Rubus suavissimus extract, sweet Hydrangea leaf extract, Fomes yucatensis extract, sesame extract, garlic extract, maca (Lepidium meyenii) extract, plant worm (Cordyceps sinensis) extract, camomile extract, and red pepper extract.

Examples of the health food material include royal jelly; dietary fiber; proteins; bifidobacteria; lactic acid bacteria; chitosan; yeast; glucosamine; lecithin; polyphenols; cartilage of animals, fish and shellfish; soft-shelled turtle; lactoferrin; freshwater clams; eicosapentaenoic acid; germanium; enzymes; creatine; carnitine; citric acid; raspberry ketone; coenzyme Q10; methylsulfonylmethane; and soybean peptides bonded with phospholipids.

An amount of the active ingredient may be determined, depending on the content of the active ingredient in the tablet described later. If necessary, two or more types of active ingredients may be used. A commercially available active ingredient may be used.

Examples of the excipient include sugars such as white soft sugar, lactose, glucose and maltose; sugar alcohols such as D-mannitol, sorbitol and maltitol; starches such as wheat starch, rice starch, potato starch and corn starch; dextrins; powdered cellulose; microcrystalline cellulose; calcium carbonate; calcium phosphate; and calcium sulfate.

An amount of the excipient may be determined, depending on the content of the active ingredient in the solid preparation described later. If necessary, two or more types of excipients may be used. A commercially available excipient may be used.

Examples of the disintegrant include low-substituted hydroxypropyl cellulose; starches such as wheat starch, rice starch, potato starch and corn starch; partly pregelatinized starch; sodium starch glycolate; carmellose; carmellose calcium; croscarmellose sodium; microcrystalline cellulose; and crospovidone.

For example, the content of hydroxypropoxy groups in the low-substituted hydroxypropyl cellulose (hereinafter, also referred to as "L-HPC") is preferably from 5 to 16% by mass, more preferably from 7 to 15% by mass, from the viewpoint of disintegration. The content of the hydroxypropoxy groups in the low-substituted hydroxypropyl cellulose may be determined by the assay described in the section "Low-substituted hydroxypropyl cellulose" of the Japanese Pharmacopoeia Seventeenth Edition.

An amount of disintegrant may be determined, depending on the content of the disintegrant in the intended solid preparation. If necessary, two or more types of disintegrants may be used. A commercially available disintegrant may be used.

PVA is a water-soluble polymer and used as a binder. Generally, PVA is produced by polymerizing vinyl acetate monomers and then subjecting the obtained polymer to saponification with an alkali. The saponification degree of PVA is from 60 to 77 mol%, preferably from 60 to 74 mol%, more preferably from 60 to 72 mol%, and particularly preferably 62 to 72 mol%. When the saponification degree is less than 60 mol%, solubility in water decreases, thereby causing phase separation and precipitation, or the compressibility becomes insufficient. In addition, when the amount is more than 77 mol%, disintegration becomes insufficient when used in a solid preparation.

The saponification degree of PVA may be measured in accordance with the saponification degree measuring method described in JIS K6726.

The viscosity of a 4% by mass aqueous solution of PVA at 20°C is from 1.5 to 100.0 mPa·s, preferably from 2.0 to 80.0 mPa·s, more preferably from 3.0 to 50.0 mPa·s. When the viscosity is less than 1.5 mPa·s, sufficient binding strength cannot be obtained. When the viscosity is more than 100mPa·s, granulation progresses to an excessive degree to generate agglomerates, or disintegration becomes insufficient when used in a solid preparation.

The viscosity at 20°C of a 4% by mass aqueous solution of PVA may be measured in accordance with the viscosity measuring method described in JIS K6726.

The content of PVA in the granulated product is from 0.1 to 5.0% by mass, preferably from 0.2 to 4.0% by mass, and more preferably from 0.5 to 3.0% by mass. When the content is less than 0.1% by mass, sufficient binding strength cannot be obtained. When the content is more than 5.0% by mass, granulation progresses to an excessive degree to generate agglomerates, or disintegration becomes insufficient when used in solid preparations.

The granulated product may comprise an optional binder excluding PVA, and an optional other additive (hereinafter, also referred to as "other additive") excluding an active ingredient, an an excipient and a disintegrant.

Examples of the optional binder excluding PVA includes hydroxypropyl cellulose, polyvinylpyrrolidone, hydroxypropyl methyl cellulose, methyl cellulose, carboxymethylcellulose sodium, a polyvinyl alcohol-acrylic acid-methyl methacrylate copolymer, and a vinyl acetate resin-vinylpyrrolidone copolymer.

Examples of the optional other additive include a flavoring agent, a sweetener, a fluidizing agent, and a dissolution aid for an active ingredient.

Examples of the flavoring agent include menthol, peppermint oil, and vanillin.

Examples of the sweetener include aspartame, acesulfame potassium, sucralose, stevia, and thaumatin.

Examples of the fluidizing agent include light anhydrous silicic acid, hydrated silicon dioxide, and magnesium aluminometasilicate.

Examples of the dissolution aid of the active ingredient include organic acids such as fumaric acid, succinic acid, malic acid, tartaric acid and adipic acid; and alkali metal salts of the organic acids.

If necessary, two or more types of the other additive may be used. A commercial other additive may be used. An amount of the other additive to be used will be described below as the content in the solid preparation.

The mean particle size of the granulated product varies depending on the application. It is preferably from 60 to 300 µm, more preferably from 70 to 200 µm, and still more preferably from 80 to 150 µm, from the viewpoint of tabletability and mass variation between tablets when used for tablets.

The mean particle size of the granulated product may be determined by using a dry method based on the Fraunhofer diffraction theory with a laser diffraction type particle size distribution measuring apparatus (Master Sizer 3000 produced by Malvern) under the conditions of dispersion pressure of 2 bar and scattering intensity of 2 to 10%, where a value corresponding to a 50% cumulative size in the volume-based cumulative particle size distribution curve is used as the mean particle size.

### (2) Method for Producing Granulated product

First, there will be described a method for producing a granulated product comprising at least one selected from an active ingredient, an excipient and a disintegrant; and 0.1 to 5.0% by mass, based on mass of the granulated product, of a polyvinyl alcohol having a saponification degree of from 60 to 77 mol% as a binder.

The granulated product may be produced, for example, by a method (hereinafter, also described as "Method A for producing a granulated product") comprising a step of granulating, while adding a second component comprising water and PVA having a saponification degree of from 60 to 77 mol%, a first component comprising at least one selected from the group consisting of an active ingredient, an excipient and a disintegrant to obtain a granulated product.

The granulated product may be produced by a method (hereinafter, also referred to as "Method B for producing a granulated product") comprising a step of granulating, while adding a second component comprising water, a first component comprising PVA having a saponification degree of from 60 to 77 mol%, and at least one selected from the group consisting of an active ingredient, an excipient and a disintegrant.

Further, the granulated product may be produced by a method (hereinafter, also referred to as "Method C for producing a granulated product") comprising a step of granulating, while adding a second component comprising water and PVA having a saponification degree of from 60 to 77 mol%, a first component comprising PVA having a saponification degree of from 60 to 77 mol% and at least one selected from the group consisting of an active ingredient, an excipient and a disintegrant.

The first component in the Method A for producing a granulated product comprises at least one selected from a group consisting of an active ingredient, an excipient and a disintegrant. The first component in the Method B or Method C for producing a granulated product comprises PVA and at least one selected from the group consisting of an active ingredient, an excipient and a disintegrant.

The second component in the Method A or Method C for producing a granulated product is an aqueous composition comprising PVA and water. The second component in the Method B for producing a granulated product comprises water.

When PVA is used in the second component, the mean particle size of the PVA is not particularly limited. It is preferably from 20 to 5000 µm, more preferably from 50 to 2000 µm, from the viewpoint of the dissolution rate during preparation of the PVA-containing aqueous composition. When PVA is used in the first component, the mean particle size of the PVA is preferably from 10 to 200 µm, more preferably from 20 to 150 µm, from the viewpoint of solubility during the granulation.

The mean particle size of PVA may be determined by using a dry method based on the Fraunhofer diffraction theory with a laser diffraction type particle size distribution measuring apparatus (Master Sizer 3000 produced by Malvern) under the conditions of a dispersion pressure of 2bar and scattering intensity of 2 to 10%, where a diameter corresponding to a 50% cumulative value in the volume-based cumulative particle size distribution curve is used as the mean particle size of PVA. Alternatively, sieving may be carried out in accordance with the dry mechanical sieving method described in JIS Z 8815:1994 (Test sieving-General requirements). The cumulative residue-on-seive percentages may be plotted by using a Rosin-Rammler diagram, and the particle size, when the cumulative residue-on-sieve percentage is 50%, may be determined as the mean particle size. When the mean particle size is 1000 µm or less, it is preferable to measure the mean particle size by using a laser diffractive particle size distribution measuring apparatus. When the mean particle size is more than 1000 µm, it is preferable to measure the mean particle size in accordance with JIS Z 8815:1994.

Examples of the water include purified water.

An amount of the water is not particularly limited. When a granulator (e.g., a fluidized bed granulator) capable of simultaneous spraying and drying is used, an amount of the water is preferably from 10 to 500 parts by mass, more preferably from 20 to 200 parts by mass, relative to 100 parts by mass of the granulated product including the first and second components and excluding the water, from the viewpoint of productivity and operability.

When a granulator (e.g., a wet stirring granulator) incapable of simultaneous spraying and drying is used, an amount of the water is preferably from 5 to 100 parts by mass, more preferably from 10 to 50 parts by mass, relative to 100 parts by mass of the granulated product including the first and second components and excluding the water, from the viewpoint of granularity.

The second component may optionally comprise an active ingredient, an excipient, and/or a disintegrant, regardless of the composition of the first component. Either one or both of the first and second components may comprise an optional other binder excluding PVA, and/or an optional other additive (hereinafter, also referred to as "other additive") excluding an active ingredient, an excipient and a disintegrant.

The granulation may be carried out by using a granulator. Examples of the granulator include a fluidized bed granulator, a stirring granulator, a tumbling fluidized bed granulator, and a spray drying granulator.

When a granulation operation in the Method A for producing a granulated product is explained by using a fluidized bed granulator as an example, a granulated product may be obtained by placing a first component in powder form comprising at least one selected from the group consisting of an active ingredient, an excipient and a disintegrant in a fluidized bed granulator, and then granulating the first component, while adding (preferably spraying) an aqueous composition comprising a predetermined PVA and water as a second component.

When a granulation operation in the Method B for producing a granulated product is explained by using a wet stirring granulator as an example, a granulated product may be obtained by placing a predetermined PVA and at least one selected from the group consisting of an active ingredient, an excipient and a disintegrant as a first component in a wet stirring granulator, and then granulating the first component, while adding (preferably spraying) a second component comprising water.

When the obtained granulated product is dried in a granulator (e.g., a fluidized bed granulator) capable of simultaneous spraying and drying, further drying is not necessary. When the obtained granulated product is not dried in such a granulator, or the granulated product is obtained in a granulator (e.g., a wet stirring granulator) incapable of simultaneous spraying and drying, drying is preferably done by a known method.

The drying may be done by using a dryer (e.g., drying oven). Examples of the dryer include a fluidized bed dryer, a flash dryer, a box type dryer, a vibration dryer, a natural convection type constant temperature dryer, a forced convection type constant temperature dryer, and a forced convection type constant temperature and constant humidity dryer. The drying temperature is preferably from 40 to 120°C.

The water content of the dried granulated product is preferably 5.0% by mass or less, more preferably 2.0% by mass or less, from the viewpoint of tablet stability. The water content of the granulated product may be determined by using a heating and drying method moisture analyzer (MX-50 produced by A&D Company Ltd.) under the conditions of 5 g of granulated product, a heating temperature of 105°C, and a heating time of 60 minutes.

### (3) Solid Preparation

The solid preparation comprises the above-described granulated product and, in a case where the granulated product does not contain an active ingredient, the active ingredient. Examples of the solid preparation include a tablet, granules and a capsule.

The content of the active ingredient in the solid preparation or in the granulated product is not particularly limited. It is preferably from 0.01 to 99.40% by mass from the viewpoint of drug effect or efficacy.

The content of the excipient in the solid preparation or in the granulated product is not particularly limited. It is preferably from 0.00 to 99.39% by mass, more preferably from 0.00 to 95.00% by mass, from the viewpoint of controlling the hardness and the disintegration time of the tablet.

The content of the disintegrant in the solid preparation or in the granulated product is preferably from 0.5 to 30.0% by mass, more preferably from 1.0 to 20.0% by mass, and still more preferably from 2.0 to 10.0% by mass, from the viewpoint of disintegration and storage stability.

The content of the other binder excluding PVA in the solid preparation or in the granulated product is preferably from 0 to 10% by mass, more preferably from 0 to 5% by mass, from the viewpoint of controlling the hardness and the disintegration time of the tablet.

The content of the other additive in the solid preparation or in the granulated product is not particularly limited. It is preferably from 0 to 10% by mass from the viewpoint of controlling the flavor of the tablet, controlling the flowability of the powder for tableting, or controlling the dissolution of the active ingredient.

The active ingredient, excipient, disintegrant, binder and/or additive may be included in either or both of the powdery first and second components in the range of each content in the tablet described above; or may be included by optionally mixing the obtained granulated product with the active ingredient, the excipient, the disintegrant, the binder and/or the additive. The binder may be included not only in an aqueous composition but also in a powder composition, before the granulation.

A tablet will be described as an example of the solid preparation.

The tablet diameter is preferably from 6 to 12 mm from the viewpoint of handling and administration.

The tablet weight is preferably from 70 to 700 mg per tablet.

The hardness of the tablet is preferably from 60 to 300N, more preferably from 80 to 250N, and still more preferably from 100 to 200N, from the viewpoint of prevention of cracking, chipping or the like when filling, transporting or removing the tablet from the PTP (press through pack) sheet. The hardness of the tablet may be measured by using a tablet hardness tester (TBH-125 produced by ERWEKA GmbH) and be obtained as the maximum breaking strength when load is applied to the tablet in the diameter direction at a rate of 1 mm/sec until the tablet breaks.

The disintegration time of the tablet is preferably within 130 seconds, more preferably within 100 seconds, and still more preferably within 80 seconds from the viewpoint of development of drug effect. The disintegration time of tablet may be determined by using a disintegration tester (NT-400 produced by Toyama Sangyo Co., Ltd.) in accordance with to the DisintegrationTest (test solution: water, no supplementary plate) of the Japanese Pharmacopoeia Seventeenth Edition.

Examples of granules or a capsule as a solid preparation preferably include those satisfying the dissolution test of the Japanese Pharmacopoeia Seventeenth Edition from the viewpoint of development of drug effect.

### (4) Method for Producing Solid Preparation

Next, there is described a method for producing a solid preparation comprising a granulated product and, in a case where the granulated product does not contain an active ingredient, the active ingredient. A method in which a tablet as a solid preparation is produced by using the obtained granulated product will be described.

When the granulated product contains no active ingredient, the granulated product is mixed with an active ingredient. At this time, the granulated product may be optionally mixed with, for example, an excipient, a disintegrant, a binder or the other additive. It may be mixed with a lubricant when an internal lubrication method is used.

The mixing method is not particularly limited. The mixing may be carried out by using a mixer. Examples of the mixer include a V-type mixer, a ribbon mixer, a container mixer, and a tumbler mixer.

A method for producing a tablet will be described as an example of the method for producing a solid preparation.

A tablet may be produced by a method comprising at least steps of: adding at least a lubricant to the granulated product to obtain a mixture (internal lubrication method), and tableting the mixture. It is also possible to produce a tablet, without adding a lubricant to the granulated product, by placing a lubricant in accordance with the external lubrication method described below, and tableting.

If necessary, the granulated product may be converted into powder for tableting. The powder for tableting may be obtained through a step of mixing the granulated product with at least one selected from the group consisting of an active ingredient, an excipient, a disintegrant, a binder, an additive and a below-mentioned lubricant. The mixing method is not particularly limited. The mixing may be done by using a mixer. Examples of the mixer include a V-type mixer, a ribbon mixer, a container mixer, and a tumbler mixer.

When there is nothing to be added to the granulated product, the granulated product itself may be used as powder for tableting.

The tableting may be carried out by using a tableting machine. Examples of the tableting machine include a rotary tableting machine and a single-punch tableting machine.

As a method of adding a lubricant (hereinafter also referred to as "lubrication method"), an internal lubrication method or an external lubrication method may be selected.

According to the internal lubrication method, lubricant-containing powder is tableted with a tableting machine equipped with a punch and a die, where a lubricant is absent at powder-contact parts of both of the punch and the die. On the other hand, according to the external lubrication method, lubricant-free powder or granulated product is tableted with a tableting machine equipped with a punch and a die, where a lubricant is present at a powder (or granulated product) contact part of the punch and/or a powder (or granulated product) contact part of the die.

When a tablet is produced with a tableting machine such as a rotary tableting machine or a single-punch tableting machine by the internal lubrication method, luricant-containing powder may be placed in a lubricant-free die and pressed by lubricant-free upper and lower punches at a predetermined pressure.

When a tablet is produced with a rotary tableting machine or the like connected to an external lubrication spray system (ELS-P1, produced by Kikusui Seisakusho Ltd.) by the external lubrication method, lubricant-free powder or granulated product may be placed in a die and pressed by upper and lower punches at a predetermined pressure, where a lubricant is present at each powder (or granulated product) contact part of the die and the punches. Examples of a method of placing a lubricant at a powder (or granulated product) contact part of the punch and/or a powder (or granulated product) contact part of the die include spraying or coating.

Examples of the lubricant include talc; magnesium stearate; calcium stearate; sodium stearyl fumarate; sucrose fatty acid esters; waxes such as paraffin wax and carnauba wax; and hardened oils such as hardened castor oil, hardened rapeseed oil and hardened beef tallow oil.

An amount of the lubricant in the internal lubrication method is preferably from 0.2 to 5.0 parts by mass, more preferably from 0.4 to 3.0 parts by mass, relative to 100 parts by mass of the lubricant-free tablet (i.e., granulated product or powder for tableting), from the viewpoint of suppression of tableting failure, delay of disintegration, and decrease of tablet hardness.

An amount of lubricant in an external lubricating method is preferably from 0.01 to 2.0 parts by mass, more preferably from 0.05 to 1.0 parts by mass, relative to 100 parts by mass of the lubricant-free tablet (i.e., granulated product or powder for tableting), from the viewpoint of suppression of tableting failure, and prevention of delay of disintegration and decrease of tablet hardness.

The tableting pressure is preferably from 20 to 400 MPa from the viewpoint of tablet hardness, disintegration time and suppression of tableting failure.

Examples of a method for producing granules include the same examples of the above-described method for producing the granulated product. The obtained granulated product is used as granules without tableting.

The method for producing a capsule comprises, for example, each step contained by the above-described method for producing a granulated product and a step of filling the obtained granulated product into a capsule.

### EXAMPLES

The invention will be explained with reference to Examples and Comparative Examples. It should not be construed that the invention is limited to or by Examples.

### <Production of PVA Powder A>

A commercially available polyvinyl acetate resin (JMR-30LL, produced by JAPAN VAM & POVAL CO., LTD.) was vacuum-dried at 100°C to remove moisture, and then dissolved in methanol to obtain a 51% by mass methanol solution of the polyvinyl acetate resin. The saponification reaction was carried out by warming 250g (500 parts by mass) of the methanol solution to 40°C; adding 6.5g (13 parts by mass) of a 3% by mass sodium hydroxide solution of 35°C in methanol thereto; sufficiently stirring the resultant mixture by using a propeller type stirring blade until the mixture becomes uniform; and then leaving the uniform mixture to stand at 40°C for 40 minutes. The obtained gel was pulverized; immersed in a mixed solvent consisting of 325 g (650 parts by mass) of methanol, 165 g (330 parts by mass) of methyl acetate and 8.5 g (17 parts by mass) of water; slowly stirred; and then subjected to addition of a 1% by mass aqueous acetic acid solution in such an amount to make the pH value of the resultant mixture to be 8 to 9 as the neutralization. After the neutralization, the solid matter and the liquid were separated, and the solid matter was dried at 60°C for 8 hours to obtain PVA particulates.

Subsequently, the PVA particulates were pulverized using a wander blender (WB-1, produced by Osaka Chemical Co., Ltd.), and then sieved through a sieve for JIS test having an opening of 150 µm to obtain PVA powder A.

A saponification degree of the obtained PVA powder A and a viscosity at 20°C of the 4% by mass aqueous solution thereof were measured in accordance with the saponification degree measuring method and the viscosity measuring method described in JIS K6726. A mean particle size thereof was also measured by using a laser diffraction type particle size distribution measuring apparatus. The results are shown in Table 1.

### <Production of PVA Powder B>

PVA powder B was produced in the same manner as in the production of PVA powder A except that an amount of the 3% by mass sodium hydroxide solution of 35°C in methanol was changed to 5.5 g (11 parts by mass).

A degree of saponification of the obtained PVA powder B, a viscosity at 20°C of the 4% by mass aqueous solution thereof and a mean particle size thereof were measured in the same manner as those of the PVA powder A. The results are shown in Table 1.

### <Preparation of PVA Powder C>

PVA powder C was produced in the same manner as in the production of PVA powder A except that an amount of the 3% by mass sodium hydroxide solution of 35°C in methanol was changed to 5.0 g (10 parts by mass).

A degree of saponification of the obtained PVA powder C, a viscosity at 20°C of the 4% by mass aqueous solution thereof and a mean particle size thereof were measured in the same manner as those of the PVA powder A. The results are shown in Table 1.

### <Preparation of PVA Powder D>

PVA powder D was produced in the same manner as in the production of PVA powder A except that an amount of the 3% by mass sodium hydroxide solution of 35°C in methanol was changed to 4.5 g (9 parts by mass).

A degree of saponification of the obtained PVA powder D, a viscosity at 20°C of the 4% by mass aqueous solution thereof and a mean particle size thereof were measured in the same manner as those of the PVA powder A. The results are shown in Table 1.

### <Preparation of PVA Powder E>

PVA powder E was produced in the same manner as in the production of PVA powder A except that an amount of the 3% by mass sodium hydroxide solution of 35°C in methanol was changed to 4.0 g (8 parts by mass).

A degree of saponification of the obtained PVA powder E, a viscosity at 20°C of the 4% by mass aqueous solution thereof and a mean particle size thereof were measured in the same manner as those of PVA powder A. The results are shown in Table 1.

### <Preparation of PVA Powder F>

PVA powder F was produced in the same manner as in the production of PVA powder A except that an amount of the 3% by mass sodium hydroxide solution of 35°C in methanol was changed to 3.5 g (7 parts by mass).

A degree of saponification of the obtained PVA powder F, a viscosity at 20°C of the 4% by mass aqueous solution thereof and a mean particle size thereof were measured in the same manner as those of the PVA powder A. The results are shown in Table 1.

### <Preparation of PVA Powder G>

PVA powder G was produced in the same manner as the production of PVA powder A except that an amount of the 3% by mass sodium hydroxide solution of 35°C in methanol was changed to 2.5 g (5 parts by mass).

A degree of saponification of the obtained PVA powder G, a viscosity at 20°C of the 4% by mass aqueous solution thereof and a mean particle size thereof were measured in the same manner as those of the PVA powder A. The results are shown in Table 1.

**Table 1**

| PVA powder | saponification degree | viscosity of 4% by mass aq. solution | mean particle size |
|---|---|---|---|
| | (mol%) | (mPa·s) | (µm) |
| A | 79 | 5.0 | 96 |
| B | 76 | 5.0 | 96 |
| C | 73 | 5.0 | 104 |
| D | 70 | 5.0 | 107 |
| E | 66 | 5.3 | 111 |
| F | 63 | 3.8 | 108 |
| G | 58 | 3.0 | 115 |

### EXAMPLE 1

An aqueous composition (hereinafter, also described as "aqueous binder solution") was prepared by dissolving 6 g of PVA powder B having a saponification degree of 76 mol%, a viscosity at 20°C of 5.0 mPa·s as determined in a 4% by mass aqueous solution, and a mean particle size of 96 µm, as a binder, in 144 g of purified water.

Next, 60 g of acetaminophen (fine powder grade, produced by Yamamoto Kagaku Kogyo Co., Ltd.) as an active ingredient, 219 g of lactose (Pharmatose 200M, produced by DFE Pharma) as an excipient, and 15 g of low-substituted hydroxypropyl cellulose having a hydroxypropoxy content of 11% by mass as a disintegrant were placed in a fluidized bed granulator (MP-01, produced by Powrex Corporation), and mixed at an air flow of 0.6 to 0.7 m³/min to obtain a powder composition.

In the same granulator, granulation was carried out, while spraying the above-mentioned aqueous binder solution under the conditions of an inlet air temperature of 80°C, an outlet air temperature of 35 to 38°C, an air flow of 0.5 to 0.6 m³/min, a spray rate of 10 g/min, and a spray air pressure of 200 kPa. After completion of spraying, the mixture was dried in the same granulator under the conditions of an inlet air temperature of 80°C and an air flow of 0.5 to 0.6 m³/min until the outlet air temperature reached 45°C, and then sieved through a JIS-test sieve with an opening of 710 µm to obtain a granulated product. Components, a mean particle size and a water content of the obtained granulated product are shown in Table 2.

Next, 100 g (100 parts by mass) of the obtained granulated product was subjected to addition of 0.5 g (0.5 parts by mass) of magnesium stearate (vegetable grade, produced Taihei Chemical Industry Co., Ltd.) as a lubricant, and mixed to obtain powder for tableting.

Then, using a tabletop tableting machine (single-punch tableting machine HANDTAB-100, produced by Ichihashi Seiki Co., Ltd.) equipped with a pair of punches having a diameter of 8 mm and a curvature radius of 12 mm and a die having a hole diameter of 8 mm, 201 mg of the powder for tableting was fed and tableted at a pressure of 10.0 kN (about 199.0MPa) to produce a tablet of 201 mg as a result of the internal lubrication method. Components, tablet hardness and disintegration time of the obtained tablets are shown in Table 3.

### EXAMPLE 2

A granulated product and tablets were produced in the same manner as in Example 1 except that PVA powder C having a saponification degree of 73 mol%, a viscosity at 20°C of 5.0 mPa·s as determined in a 4% by mass aqueous solution, and a mean particle size of 104 µm, was used as a binder. Components, a mean particle size and a water content of the obtained granulated product are shown in Table 2. Components, tablet hardness and disintegration time of the obtained tablets are shown in Table 3.

### EXAMPLE 3

A granulated product and tablets were produced in the same manner as in Example 1 except that PVA powder D having a saponification degree of 70 mol%, a viscosity at 20°C of 5.0 mPa·s as determined in a 4% by mass aqueous solution, and a mean particle size of 107 µm, was used as a binder. Components, a mean particle size and a water content of the obtained granulated product are shown in Table 2. Components, tablet hardness and disintegration time of the obtained tablets are shown in Table 3.

### EXAMPLE 4

A granulated product and tablets were produced in the same manner as in Example 1 except that PVA powder E having a saponification degree of 66 mol%, a viscosity at 20°C of 5.3 mPa·s as determined in a 4% by mass aqueous solution, and a mean particle size of 111 µm, was used as a binder. Components, a mean particle size and a water content of the obtained granulated product are shown in Table 2. Components, tablet hardness and disintegration time of the obtained tablets are shown in Table 3.

### EXAMPLE 5

A granulated product and tablets were produced in the same manner as in Example 1 except that PVA powder F having a saponification degree of 63 mol%, a viscosity at 20°C of 3.8 mPa·s as determined in a 4% by mass aqueous solution, and a mean particle size of 108 µm, was used as a binder. Components, a mean particle size and a water content of the obtained granulated product are shown in Table 2. Components, tablet hardness and disintegration time of the obtained tablets are shown in Table 3.

### EXAMPLE 6

A granulated product and tablets were produced in the same manner as in Example 1 except that 3 g of PVA powder D as a binder was dissolved in 147 g of purified water to prepare an aqueous binder solution, and the amount of lactose placed in the fluidized bed granulator was changed to 222 g. Components, a mean particle size and a water content of the obtained granulated product are shown in Table 2. Components, tablet hardness and disintegration time of the obtained tablets are shown in Table 3.

### EXAMPLE 7

A granulated product and tablets were produced in the same manner as in Example 1 except that 1.5 g of PVA powder D as a binder was dissolved in 148.5 g of purified water to prepare an aqueous binder solution, and the amount of lactose placed in the fluidized bed granulator was changed to 223.5 g. Components, a mean particle size and a water content of the obtained granulated product are shown in Table 2. Components, tablet hardness and disintegration time of the obtained tablets are shown in Table 3.

### EXAMPLE 8

An aqueous binder solution was prepared by dissolving 6 g of PVA powder C having a saponification degree of 73 mol%, a viscosity at 20°C of 5.0 mPa·s as determined in a 4% by mass aqueous solution, and a mean particle size of 104 µm, in 144 g of purified water.

Next, 60 g of acetaminophen (fine powder grade produced by Yamamoto Kagaku Kogyo Co., Ltd.) as an active ingredient and 219 g of D-mannitol (PEARLITOL 25C, produced by Roquette) as an excipient were placed in a fluidized bed granulator (MP-01, produced by Powrex Corporation), and mixed at an air flow of 0.6 to 0.7 m³/min to obtain a powder composition.

In the same granulator, granulation was carried out, while spraying the above-mentioned aqueous binder solution under the conditions of an inlet air temperature of 80°C, an outlet air temperature of 35 to 38°C, an air flow of 0.5 to 0.6 m³/min, a spray rate of 10 g/min, and a spray air pressure of 200 kPa. After completion of spraying, the mixture was dried in the same granulator under the conditions of an inlet air temperature of 80°C and an air flow of 0.5 to 0.6 m³/min until the outlet air temperature reached 45°C, and then sieved through a JIS-test sieve with an opening of 710 µm to obtain a granulated product. Components, a mean particle size and a water content of the obtained granulated product are shown in Table 2.

Next, 95 g (95 parts by mass) of the obtained granulated product and 5 g (5 parts by mass) of low-substituted hydroxypropyl cellulose having a hydroxypropoxy content of 11% by mass as a disintegrant were mixed, then subjected to addition of 0.5 g (0.5 parts by mass) of magnesium stearate (vegetable grade, produced by Taihei Chemical Industry Co., Ltd.) as a lubricant, and mixed to obtain powder for tableting.

Then, using a tabletop tableting machine (single-punch tableting machine HANDTAB-100, produced by by Ichihashi Seiki Co., Ltd.) equipped with a pair of punches having a diameter of 8 mm and a curvature radius of 12 mm and a die having a hole diameter of 8 mm, 201 mg of the powder for tableting was fed and tableted at a pressure of 7.5 kN (about 149.3 MPa) to produce a tablet of 201 mg as the internal lubrication method. Components, tablet hardness and disintegration time of the obtained tablets are shown in Table 3.

### EXAMPLE 9

A granulated product and tablets were produced in the same manner as in Example 8 except that 95 g (95 parts by mass) of the granulated product obtained in Example 8 and 5 g (5 parts by mass) of sodium starch glycolate (Primojel, produced by DFE Pharma) as a disintegrant were mixed. Components, tablet hardness and disintegration time of the obtained tablets are shown in Table 3.

### EXAMPLE 10

An aqueous binder solution was prepared by dissolving 6 g of PVA powder D having a saponification degree of 70 mol%, a viscosity at 20°C of 5.0 mPa·s as determined in a 4% by mass aqueous solution, and a mean particle size of 107 µm, as a binder, in 144 g of purified water.

Next, 279 g of D-mannitol (PEARLITOL 25C, produced by Roquette) as an excipient and 15 g of low-substituted hydroxypropyl cellulose having a hydroxypropoxy content of 11% by mass as a disintegrant were placed in a fluidized bed granulator (MP-01, produced by Powrex Corporation), and mixed at an air flow of 0.6 to 0.7 m³/min to obtain a powder composition.

In the same granulator, granulation was carried out, while spraying the above-mentioned aqueous binder solution under the conditions of an inlet air temperature of 80°C, an outlet air temperature of 35 to 38°C, an air flow of 0.5 to 0.6 m³/min, a spray rate of 10 g/min, and a spray air pressure of 200 kPa. After completion of spraying, the mixture was dried in the same granulator under the conditions of an inlet air temperature of 80°C and an air flow of 0.5 to 0.6 m³/min until the outlet air temperature reached 45°C, and then sieved through a JIS-test sieve with an opening of 710 µm to obtain a granulated product. Components, a mean particle size and a water content of the obtained granulated product are shown in Table 2.

Next, 90 g (90 parts by mass) of the obtained granulated product and 10 g (10 parts by mass) of acetaminophen (fine powder grade, produced by Yamamoto Kagaku Kogyo) as an active ingredient were mixed, then subjected to addition of 0.5 g (0.5 parts by mass) of magnesium stearate (vegetable grade, produced by Taihei Chemical Industry Co., Ltd.) as a lubricant, and mixed to obtain powder for tableting.

Then, using a tabletop tableting machine (single-punch tableting machine HANDTAB-100, produced by Ichihashi Seiki Co., Ltd.) equipped with a pair of punches having a diameter of 8 mm and a curvature radius of 12 mm and a die having a hole diameter of 8 mm, 201 mg of the powder for tableting was fed and tableted at pressure of 10.0 kN (about 199.0 MPa) to produce a tablet of 201 mg as a result of the internal lubrication method. Components, tablet hardness and disintegration time of the obtained tablets are shown in Table 3.

### EXAMPLE 11

An aqueous binder solution was prepared by dissolving 6 g of PVA powder D having a saponification degree of 70 mol%, a viscosity at 20°C of 5.0 mPa·s as determined in a 4% by mass aqueous solution, and a mean particle size of 107 µm, as a binder, in 144 g of purified water.

Next, 294 g of acetaminophen (fine powder grade, produced by Yamamoto Kagaku Kogyo) as an active ingredient was placed in a fluidized bed granulator (MP-01, produced by Powrex Corporation), and flowed at an air flow of 0.6 to 0.7 m³/min to obtain a powder composition.

In the same granulator, granulation was carried out, while spraying the above-mentioned aqueous binder solution under the conditions of an inlet air temperature of 80°C, an outlet air temperature of 35 to 38°C, an air flow of 0.5 to 0.6 m³/min, a spray rate of 10g/min, and a spray air pressure of 200 kPa. After completion of spraying, the mixture was dried in the same granulator under the conditions of an inlet air temperature of 80°C and an air flow of 0.5 to 0.6 m³/min until the outlet air temperature reached 45°C, and then sieved through a JIS-test sieve with an opening of 710 µm to obtain a granulated product. Components, a mean particle size and a water content of the obtained granulated product are shown in Table 2.

Next, 50 g (50 parts by mass) of the obtained granulated product, 38 g (38 parts by mass) of lactose (Dilactose^{®} S, produced by Freund Corporation) as an excipient, 10 g (10 parts by mass) of microcrystalline cellulose (Avicel^{®} PH-101, produced by FMC Corporation), and 2 g (2 parts by mass) of crospovidone (Kollidon^{®} CL, produced by BASF) as a disintegrant were mixed, then subjected to addition of 0.5 g (0.5 parts by mass) of magnesium stearate (vegetable grade, produced by Taihei Chemical Industry Co., Ltd.) as a lubricant, and mixed to obtain powder for tableting.

Then, using a tabletop tableting machine (single-punch tableting machine HANDTAB-100, produced by Ichihashi Seiki Co., Ltd.) with a pair of punches having a diameter of 8 mm and a curvature radius of 12 mm and a die having a hole diameter of 8 mm, 201 mg of the powder for tableting was fed and tableted at a tableting pressure of 10 kN (about 199.0 MPa) to produce a tablet of 201 mg as a result of the inner lubrication method. Components, tablet hardness and disintegration time of the obtained tablets are shown in Table 3.

### EXAMPLE 12

An aqueous binder solution was prepared by dissolving 6 g of PVA powder D having a saponification degree of 70 mol%, a viscosity at 20°C of 5.0 mPa·s as determined in a 4% by mass aqueous solution, and a mean particle size of 107 µm, as a binder, in 144 g of purified water.

Next, 205.8 g of lactose (Pharmatose 200M, produced by DFE Pharma) and 88.2 g of corn starch (Nisshoku Cornstarch W, produced by Nihon Shokuhin Kako Co., Ltd.) as excipients were placed in a fluidized bed granulator (MP-01, produced by Powrex Corporation), and mixed at an air flow of 0.6 to 0.7 m³/min to obtain a powder composition.

In the same granulator, granulation was carried out, while spraying the above-mentioned aqueous binder solution under the conditions of an inlet air temperature of 80°C, an outlet air temperature of 35 to 38°C, an air flow of 0.5 to 0.6 m³/min, a spray rate of 10 g/min, and a spray air pressure of 200 kPa. After completion of spraying, the mixture was dried in the same granulator under the conditions of an inlet air temperature of 80°C and an air flow of 0.5 to 0.6 m³/min until the outlet air temperature reached 45°C, and then sieved through a JIS-test sieve with an opening of 710 µm to obtain a granulated product. Components, a mean particle size and a water content of the obtained granulated product are shown in Table 2.

Next, 88 g (88 parts by mass) of the obtained granulated product and 10 g (10 parts by mass) of acetaminophen (fine powder grade, produced by Yamamoto Kagaku Kogyo Co., Ltd.) as an active ingredient, and 2 g (2 parts by mass) of croscarmellose sodium (Ac-Di-Sol^{®}, produced by FMC Corporation) as a disintegrant were mixed, then subjected to addition of 0.5 g (0.5 parts by mass) of magnesium stearate (vegetable grade, produced by Taihei Chemical Industry Co., Ltd.) as a lubricant, and mixed to obtain powder for tableting.

Then, using a tabletop tableting machine (single-punch tableting machine HANDTAB-100, produced by Ichibashi Seiki Co., Ltd.) equipped with a pair of punches having a diameter of 8 mm and a curvature radius of 12 mm and a die having a hole diameter of 8 mm, 201 mg of the powder for tableting was fed and tableted at a pressure of 10kN (about 199.0MPa) to produce a tablet of 201 mg as a result of the inner lubrication method. Components, tablet hardness and disintegration time of the obtained tablets are shown in Table 3.

### EXAMPLE 13

A powder composition was obtained by mixing 60 g of acetaminophen (fine powder grade, produced by Yamamoto Kagaku kogyo Co., Ltd.) as an active ingredient, 210 g of lactose (Pharmatose 200M, produced by DFE Pharma) as an excipient, 15 g of low-substituted hydroxypropyl cellulose having a hydroxypropoxy content of 11% by mass as a disintegrant and 15 g of PVA powder D at a main blade rotation speed of 450 rpm and a cross screw rotation speed of 3000 rpm for 1 minute in a wet stirring granulator (VG-05, produced by Powrex Corporation).

Subsequently, in the same granulator, the powder composition was subjected to addition of 54 g of water, kneaded at a main blade rotation speed of 450 rpm and a cross screw rotation speed of 3000 rpm for 5 minutes, and then sieved through a JIS-test sieve with an opening of 1 mm to obtain a wet granulated product. The wet granulated product was placed in a fluidized bed granulation dryer (Multiplex MP-01, produced by Powrex Corporation), dried under the conditions of an inlet air temperature of 80°C and an air flow of 0.6 to 0.8 m³/min until the outlet air temperature reached 45°C, and sieved through a JIS-test sieve with an opening of 710 µm to obtain a granulated product. Components, a mean particle size and a water content of the obtained granulated product are shown in Table 2.

Next, 100 g (100 parts by mass) of the obtained granulated product was subjected to addition of 0.5 g (0.5 parts by mass) of magnesium stearate (vegetable grade, produced by Taihei Chemical Industry Co., Ltd.) as a lubricant, and mixed to obtain powder for tableting.

Then, using a tabletop tableting machine (single-punch tableting machine HANDTAB-100, produced by Ichihashi Seiki Co., Ltd.) equipped with a pair of punches having a diameter of 8 mm and a curvature radius of 12 mm and a die having a hole diameter of 8 mm, 201 mg of the powder for tableting was fed and tableted at a pressure of 15.0 kN (about 298.6 MPa) to produce a tablet of 201 mg as a result of the internal lubrication method. Components, tablet hardness and disintegration time of the obtained tablets are shown in Table 3.

### EXAMPLE 14

A granulated product and tablets were produced in the same manner as in Example 13 except that 216 g of lactose (Pharmatose 200M, produced by DFE Pharma) as an excipient and 9 g of PVA powder E as a binder were used. Components, a mean particle size and a water content of the obtained granulated product are shown in Table 2. Components, tablet hardness and disintegration time of the obtained tablets are shown in Table 3.

### EXAMPLE 15

A granulated product and tablets were produced in the same manner as in Example 14 except that PVA powder F was used as a binder. Components, a mean particle size and a water content of the obtained granulated product are shown in Table 2. Components, tablet hardness and disintegration time of the obtained tablets are shown in Table 3.

### Comparative Example 1

A granulated product and tablets were produced in the same manner as in Example 1 except that PVA powder A having a saponification degree of 79 mol%, a viscosity at 20°C of 5.0 mPa·s as determined in a 4% by mass aqueous solution, and a mean particle size of 96 µm was used as a binder. Components, a mean particle size and a water content of the obtained granulated product are shown in Table 2. Components, tablet hardness and disintegration time of the obtained tablets are shown in Table 3.

### Comparative Example 2

Granulation was carried out in the same manner as in Example 1 except that PVA powder G having a saponification degree of 58 mol%, a viscosity at 20°C of 3.0 mPa·s as determined in a 4% by mass aqueous solution, and a mean particle size of 115 µm, was used as a binder. As a result, the gel-like precipitate of PVA, which appeared as phase separation, clogged the spray nozzle and made it difficult to continue to spray, thereby causing the granulation operation to be stopped.

### Comparative Example 3

Granulation was carried out in the same manner as in Example 1 except that 18 g of PVA powder D was dissolved as a binder in 132 g of purified water to prepare an aqueous binder solution, and the amount of lactose placed in the fluidized bed granulator was changed to 207 g. As a result, granulation progressed to an excessive degree so that the flow state deteriorated, and blocking occurred in the fluidized bed granulator, thereby causing the granulation operation to be stopped.

### Comparative Example 4

A granulated product and tablets were produced in the same manner as in Example 8 except that PVA powder A was used as a binder. Components, a mean particle size and a water content of the obtained granulated product are shown in Table 2. Components, tablet hardness and disintegration time of the obtained tablets are shown in Table 3.

### Comparative Example 5

A granulated product and tablets were produced in the same manner as in Example 9 except that PVA powder A was used as a binder. Components, a mean particle size and a water content of the obtained granulated product are shown in Table 2. Components, tablet hardness and disintegration time of the obtained tablets are shown in Table 3.

### Comparative Example 6

A granulated product and tablets were produced in the same manner as in Example 14 except that PVA powder A was used as a binder. Components, a mean particle size and a water content of the obtained granulated product are shown in Table 2. Components, tablet hardness and disintegration time of the obtained tablets are shown in Table 3.

### Comparative Example 7

A granulated product and tablets were produced in the same manner as in Example 13 except that the amount of lactose placed in the wet stirring granulator was changed to 225 g and no PVA powder was used. Components, a mean particle size and a water content of the obtained granulated product are shown in Table 2. Components, tablet hardness and disintegration time of the obtained tablets are shown in Table 3.

**Table 2**

| | type of granulation - | active ingredient | | excipient | | | disintegrant | binder | | | properties of granulated prod. | |
|---|---|---|---|---|---|---|---|---|---|---|---|---|
| | | acetaminophen | ethenzamide | lactose | D-mannitol | corn starch | L-HPC | PVA | saponification degree of PVA | addition form | mean particle size | water content |
| | | (mass%) | (mass%) | (mass%) | (mass%) | (mass%) | (mass%) | (mass%) | (mol%) | - | (µm) | (%) |
| Example1 | fluidized bed | 20,0 | 0.0 | 73.0 | 0.0 | 0.0 | 5.0 | 2.0 | 76 | solution | 122 | 1.0 |
| Example2 | fluidized bed | 20.0 | 0.0 | 73.0 | 0.0 | 0.0 | 5.0 | 2.0 | 73 | solution | 122 | 1.1 |
| Example3 | fluidized bed | 20.0 | 0.0 | 73.0 | 0.0 | 0.0 | 5.0 | 2.0 | 70 | solution | 115 | 1.1 |
| Example4 | fluidized bed | 20.0 | 0.0 | 73.0 | 0.0 | 0.0 | 5.0 | 2.0 | 66 | solution | 117 | 1.2 |
| Example5 | fluidized bed | 20.0 | 0.0 | 73.0 | 0.0 | 0.0 | 5.0 | 2.0 | 63 | solution | 89 | 0.8 |
| Example6 | fluidized bed | 20.0 | 0.0 | 74.0 | 0.0 | 0.0 | 5.0 | 1.0 | 70 | solution | 83 | 0.9 |
| Example7 | fluidized bed | 20.0 | 0.0 | 74.5 | 0.0 | 0.0 | 5.0 | 0.5 | 70 | solution | 64 | 0.7 |
| Example8 | fluidized bed | 20.0 | 0.0 | 0.0 | 73.0 | 0.0 | 0.0 | 2.0 | 73 | solution | 70 | 0.4 |
| Example10 | fluidized bed | 0.0 | 0.0 | 0.0 | 93.0 | 0.0 | 5.0 | 2.0 | 70 | solution | 77 | 0.3 |
| Example11 | fluidized bed | 98.0 | 0.0 | 0.0 | 0.0 | 0.0 | 0.0 | 2.0 | 70 | solution | 110 | 0.3 |
| Example12 | fluidized bed | 0.0 | 0.0 | 68.6 | 0.0 | 29.4 | 0.0 | 2.0 | 70 | solution | 92 | 1.5 |
| Example13 | wet stirring | 20.0 | 0.0 | 70.0 | 0.0 | 0.0 | 5.0 | 5.0 | 70 | powder | 188 | 1.0 |
| Example14 | wet stirring | 20.0 | 0.0 | 72.0 | 0.0 | 0.0 | 5.0 | 3.0 | 66 | powder | 108 | 0.4 |
| Example15 | wet stirring | 20.0 | 0.0 | 72.0 | 0.0 | 0.0 | 5.0 | 3.0 | 63 | powder | 100 | 0.5 |
| Comp.Ex.1 | fluidized bed | 20.0 | 0.0 | 73.0 | 0.0 | 0.0 | 5.0 | 2.0 | 79 | solution | 111 | 1.2 |
| Comp.Ex.2 | fluidized bed | 20.0 | 0.0 | 73.0 | 0.0 | 0.0 | 5.0 | 2.0 | 58 | solution | - | - |
| Comp.Ex.3 | fluidized bed | 20.0 | 0.0 | 69.0 | 0.0 | 0.0 | 5.0 | 6.0 | 70 | solution | - | - |
| Comp.Ex.4 | fluidized bed | 20.0 | 0.0 | 0.0 | 73.0 | 0.0 | 0.0 | 2.0 | 79 | solution | 75 | 0.3 |
| Comp.Ex.6 | wet stirring | 20.0 | 0.0 | 72.0 | 0.0 | 0.0 | 5.0 | 3.0 | 79 | powder | 124 | 0.7 |
| Comp.Ex.7 | wet stirring | 20.0 | 0.0 | 75.0 | 0.0 | 0.0 | 5.0 | 0.0 | - | none | 57 | 0.6 |

**Table 3**

| | granulated product | active ingredient | excipient | | disintegrant | | | | lubricant | tableting pressure | tablet properties | |
|---|---|---|---|---|---|---|---|---|---|---|---|---|
| | | acetaminophen | lactose | microcrystalline cellulose | L-HPC | sodium starch glycolate | croscarmellose sodium | crospovidon | magnesium stearate | | hardness | disintegration time |
| | (pbm) | (part by mass: pbm) | (pbm) | (pbm) | (pbm) | (pbm) | (pbm) | (pbm) | (pbm) | (kN) | (N) | (second) |
| Example1 | 100.0 | 0.0 | 0.0 | 0.0 | 0.0 | 0.0 | 0.0 | 0.0 | 0.5 | 10.0 | 132 | 115 |
| Example2 | 100.0 | 0.0 | 0.0 | 0.0 | 0.0 | 0.0 | 0.0 | 0.0 | 0.5 | 10.0 | 127 | 89 |
| Example3 | 100.0 | 0.0 | 0.0 | 0.0 | 0.0 | 0.0 | 0.0 | 0.0 | 0.5 | 10.0 | 134 | 84 |
| Example4 | 100.0 | 0.0 | 0.0 | 0.0 | 0.0 | 0.0 | 0.0 | 0.0 | 0.5 | 10.0 | 133 | 64 |
| Example5 | 100.0 | 0.0 | 0.0 | 0.0 | 0.0 | 0.0 | 0.0 | 0.0 | 0.5 | 10.0 | 109 | 43 |
| Example6 | 100.0 | 0.0 | 0.0 | 0.0 | 0.0 | 0.0 | 0.0 | 0.0 | 0.5 | 10.0 | 111 | 50 |
| Example7 | 100.0 | 0.0 | 0.0 | 0.0 | 0.0 | 0.0 | 0.0 | 0.0 | 0.5 | 10.0 | 72 | 31 |
| Example8 | 95.0 | 0.0 | 0.0 | 0.0 | 5.0 | 0.0 | 0.0 | 0.0 | 0.5 | 7.5 | 128 | 71 |
| Example9 | 95.0 | 0.0 | 0.0 | 0.0 | 0.0 | 5.0 | 0.0 | 0.0 | 0.5 | 7.5 | 109 | 72 |
| Example10 | 90.0 | 10.0 | 0.0 | 0.0 | 0.0 | 0.0 | 0.0 | 0.0 | 0.5 | 10.0 | 122 | 66 |
| Example11 | 50.0 | 0.0 | 38.0 | 10.0 | 0.0 | 0.0 | 0.0 | 2.0 | 0.5 | 10.0 | 92 | 27 |
| Example12 | 88.0 | 10.0 | 0.0 | 0.0 | 0.0 | 0.0 | 2.0 | 0.0 | 0.5 | 10.0 | 81 | 127 |
| Example13 | 100.0 | 0.0 | 0.0 | 0.0 | 0.0 | 0.0 | 0.0 | 0.0 | 0.5 | 15.0 | 105 | 89 |
| Example14 | 100.0 | 0.0 | 0.0 | 0.0 | 0.0 | 0.0 | 0.0 | 0.0 | 0.5 | 15.0 | 74 | 30 |
| Example15 | 100.0 | 0.0 | 0.0 | 0.0 | 0.0 | 0.0 | 0.0 | 0.0 | 0.5 | 15.0 | 74 | 24 |
| Comp.Ex.1 | 100.0 | 0.0 | 0.0 | 0.0 | 0.0 | 0.0 | 0.0 | 0.0 | 0.5 | 10.0 | 126 | 142 |
| Comp.Ex.4 | 95.0 | 0.0 | 0.0 | 0.0 | 5.0 | 0.0 | 0.0 | 0.0 | 0.5 | 7.5 | 124 | 142 |
| Comp.Ex.5 | 95.0 | 0.0 | 0.0 | 0.0 | 0.0 | 5.0 | 0.0 | 0.0 | 0.5 | 7.5 | 110 | 152 |
| Comp.Ex.6 | 100.0 | 0.0 | 0.0 | 0.0 | 0.0 | 0.0 | 0.0 | 0.0 | 0.5 | 15.0 | 85 | 132 |
| Comp.Ex.7 | 100.0 | 0.0 | 0.0 | 0.0 | 0.0 | 0.0 | 0.0 | 0.0 | 0.5 | 15.0 | 54 | 21 |

In Examples 1 to 5, where PVA having a saponification degree of 63 to 76 mol% was used, a granulated product having a sufficient mean particle size was obtained, and a tablet having good hardness and disintegration was obtained. In addition, it was found that as the saponification degree of PVA became lower, the improvement of disintegration became greater. On the other hand, in Comparative Example 1, where PVA having a saponification degree of 79 mol% was used, granulation and tablet hardness were sufficient, but disintegration was insufficient.

PVA is a highly hydrophilic water-soluble binder having a large number of hydroxy groups in its molecule. However, it took a long time to dissolve PVA in the tablet of Comparative Example 1 and the disintegration wa insufficient. It is considered to be because the intermolecular hydrogen bonds are strong so that the rate of dissolution into water is slow. On the other hand, the tablets of Examples 1 to 5 showed the tendency that as the saponification degree became lower, the disintegration time became faster. It is considered that as the saponification degree becomes lower, the amount of the hydrophobic acetyl group bonded to the hydroxy group becomes larger, so that the intermolecular hydrogen bonds become weaker and the rate of dissolution into water increases. It is evident from the results of Comparative Example 2, where PVA having the saponification degree of 58 mol% was used, that PVA having a too low saponification degree is unsuitable as a binder. It is because the solubility in water is lowered due to an increase in hydrophobicity, and the gel-like precipitate of PVA, which appears by phase separation, clogs the spray nozzle to prevent spraying and a granulated product cannot be obtained.

It is evident from the results of Examples 3, 6 and 7, where PVA having a saponification degree of 70 mol% was used, that when an amount of PVA is smaller, a mean particle size and tablet hardness of the granulated product become lower, but have sufficient granulation and tablet hardness, and disintegration is improved. On the contrary, it is evident that when an amount of PVA is larger, granulation and tablet hardness are improved. However, it is also evident from the results of Comparative Example 3 that when an amount of PVA is too large, the granulation progresses to an excessive degree, so that the flow state during granulation is deteriorated, the blocking occurs in the fluidized bed granulator, and the granulation operation cannot be continued, thereby failing to obtain the granulated product. Thus, it is considered that there is an appropriate range for the amount of PVA to be added.

It is evident from the results of Example 8 and Comparative Example 4, where an excipient different from that of Examples 1 to 7 was used and a disintegrant was externally added after the granulation instead of the internal addition during the granulation, that the disintegration is improved by lowering the saponification degree of PVA regardless of the type of excipient and the addition method (internal addition or external addition) of the disintegrant. Further, it is evident from the results of Example 9 and Comparative Example 5, where a disintegrant different from that of Example 8 was used, that when the saponification degree of PVA is decreased, the disintegration is improved regardless of the type of disintegrant.

In all of Example 10, where the active ingredient was externally added to a granulated product containing three components: an excipient, a disintegrant and PVA, Example 11, where an excipient and a disintegrant were externally added to a granulated product containing two components: an active ingredient and PVA, and Example 12, where an active ingredient and a disintegrant were externally added to a granulated product containing two components: an excipient and PVA, tablets having good hardness and disintegration were obtained. It is evident from these results that a tablet having good properties can be obtained regardless of the addition method (internal addition or external addition) of an excipient, a disintegrant and an active ingredient.

Granulated products having sufficient particle sizes, and tablets having good hardness and disintegration were obtained also in Examples 13 to 15, where granulation was carried out by adding water to an active ingredient, an excipient, a disintegrant and PVA having a saponification degree of 70 to 63 mol%, which is different from the granulation method in Examples 1 to 7, where an aqueous PVA solution was added to the active ingredient, an excipient and a disintegrant. On the other hand, the granulated product had a sufficient mean particle size and tablet hardness, but disintegration was poor in Comparative Example 6, where PVA having a saponification degree of 79 mol% was used by the same granulation method as in Examples 13 to 15. A granulated product having a sufficient particle size could not be obtained due to insufficient binding strength, and a tablet having sufficient hardness could not be obtained in Comparative Example 7, where PVA was not added.

## Claims

1. A granulated product comprising:
at least one selected from the group consisting of an active ingredient, an excipient and a disintegrant; and
0.1 to 5.0% by mass, based on mass of the granulated product, of a polyvinyl alcohol having a saponification degree of from 60 to 77 mol%.

2. The granulated product according to claim 1, comprising the excipient selected from the group consisting of sugars, sugar alcohols, starches, dextrins, powdered cellulose, microcrystalline cellulose, calcium carbonate, calcium phosphate and calcium sulfate.

3. The granulated product according to claim 1 or claim 2, comprising the disintegrant selected from the group consisting of low-substituted hydroxypropyl cellulose, starches, partially pregelatinized starches, sodium starch glycolate, carmellose, carmellose calcium, croscarmellose sodium, microcrystalline cellulose and crospovidone.

4. A solid preparation comprising:
the granulated product claimed in any one of claims 1 to 3; and
an active ingredient in a case where the granulated product does not comprise the active ingredient.

5. The solid preparation according to claim 4, wherein the sold preparation is in a form of a tablet, granule or capsule.

6. A method for producing a granulated product, the method comprising a granulation step of granulating, while adding a second component comprising at least water, a first component comprising at least one selected from the group consisting of an active ingredient, an excipient and a disintegrant to obtain the granulated product, wherein the first component and/or the second component comprises a polyvinyl alcohol having a saponification degree of from 60 to 77 mol% in an amount of from 0.1 to 5.0 mass%, based on mass of the granulated product.

7. The method for producing a granulated product according to claim 6, comprising the excipient selected from the group consisting of sugars, sugar alcohols, starches, dextrins, powdered cellulose, microcrystalline cellulose, calcium carbonate, calcium phosphate and calcium sulfate.

8. The method for producing a granulated product according to claim 6 or claim 7, comprising the disintegrant selected from the group consisting of low-substituted hydroxypropyl cellulose, starches, partially pregelatinized starches, sodium starch glycolate, carmellose, carmellose calcium, croscarmellose sodium, microcrystalline cellulose and crospovidone.
